Europäisches Patentamt

European Patent Office

Office européen des brevets

(19)

(11) Numéro de publication: **0 017 560**
**B1**

(12)

# FASCICULE DE BREVET EUROPEEN

(45) Date de publication du fascicule de brevet:
**31.03.82**

(21) Numéro de dépôt: **80400398.6**

(22) Date de dépôt: **26.03.80**

(51) Int. Cl.³: **C 07 C 76/02, C 07 C 79/04**

(54) Procédé et installation de fabrication de nitroparaffines par nitration d'hydrocarbures en phase gazeuse.

(30) Priorité: **10.04.79 FR 7908997**

(43) Date de publication de la demande:
**15.10.80 Bulletin 80/21**

(45) Mention de la délivrance du brevet:
**31.03.82 Bulletin 82/13**

(84) Etats contractants désignés:
**AT BE CH DE FR GB IT LU NL SE**

(56) Documents cités:
**FR-A-2 158 708**
**FR-A-2 199 753**

Le dossier contient des informations techniques
présentées postérieurement au dépôt de la demande et
ne figurant pas dans le présent fascicule.

(73) Titulaire: **SOCIETE CHIMIQUE DE LA GRANDE
PAROISSE AZOTE ET PRODUITS CHIMIQUES, 8, rue
Cognacq-Jay, F-75321 Paris Cedex 07 (FR)**

(72) Inventeur: **Lhonore, Pierre, 1090, bd Jeanne d'Arc,
F-59500 Douai (FR)**
Inventeur: **Quibel, Jacques, 40 rue de la Muette,
F-78600 Maisons Laffitte (FR)**
Inventeur: **Jacquinot, Bernard, 59, rue Léon Bathiat,
F-59500 Douai (FR)**

(74) Mandataire: **Bouton Neuvy, Liliane et al, L'Air liquide,
Société Anonyme pour L'Etude et L'Exploitation des
Procédés Georges Claude 75, Quai d'Orsay,
F-75321 Paris Cedex 07 (FR)**

Procédé et installation de fabrication de nitroparaffines par nitration d'hydrocarbures
en phase gazeuse

La présente invention concerne un procédé de fabrication de nitroparaffines par nitration en phase gazeuse homogène d'un hydrocarbure saturé inférieur à $C_5$ par un agent nitrant, et l'installation pour mettre en œuvre cette nitration.

On connaît déjà divers procédé de nitration, notamment de l'éthane, du propane et de leurs mélanges en phase gazeuse, tels ceux décrits dans les demandes de brevets européennes publiées sous les numéros 4812 et 11 553 au nom de la Société Chimique de la Grande Paroisse. Selon ces procédés les rapports quantitatifs des divers constituants du mélange réactionnel, les temps de contact réactionnel, les températures, pressions réactionnelles sont choisies et contrôlés de manière telle que la nitration de l'hydrocarbure ou des mélanges de ceux-ci soit mise en œuvre en phase gazeuse homogène, et en fonction du spectre attendu des nitroparaffines.

L'un des problèmes industriels actuels réside dans la recherche des moyens permettant d'obtenir un pourcentage des différentes nitroparaffines adapté aux exigences du marché. Or, il s'avère que le 2-nitropropane ne représente pas en cette période la nitroparaffine la plus valorisable; par contre les besoins en nitrométhane sont devenus importants en fonction des usages croissants de ce produit.

L'augmentation de la température de réaction permet une production proportionnellement plus élevée en nitrométhane mais au détriment du prix de revient, conséquence d'une dégradation plus poussée des hydrocarbures en produits oxydés.

Il a été trouvé un procédé qui pallie en grande partie cet inconvénient tout en permettant une production en accord avec la demande du marché et à prix de revient avantageux pour les nitroparaffines fabriquées.

Selon le procédé de l'invention on met en œuvre la réaction de nitration en boucle fermée sous pression avec séparation des effluents réactionnels en phase gazeuse et en phase liquide; on extrait la phase liquide contenant notamment les nitroparaffines produites et on la traite en vue de la récupération de celles-ci; on soumet la phase gazeuse à un traitement primaire d'épuration, et on recycle dans la boucle de nitration la majeure partie de la phase gazeuse épurée et réchauffée sous la pression de la dite boucle; on procède à une purge continue de déconcentration de l'autre partie de la phase gazeuse accompagnée d'opérations d'épuration et de récupération de l'hydrocarbure et des oxydes d'azote; on fait un appoint continu en hydrocarbure d'une part et en agent nitrant d'autre part.

La pression dans la boucle de nitration est comprise entre 1 bar et 30 bars, et de préférence en 8 et 20 bars.

Pour limiter le phénomène de dégradation poussée de l'hydrocarbure en produits oxydés on a intérêt à maintenir la température de réaction à un niveau déterminé avec une parfaite maîtrise de la courbe l'élévation de température au cours de la nitration, en conduisant celle-ci dans un réacteur adapté pour un bon échange de chaleur et permettant de conduire la nitration en régime thermique progressif et régulier avec une température de sortie des effluents en fin de nitration inférieure à 400°C.

Le procédé convient particulièrement bien à la fabrication en phase gazeuse homogène de nitroparaffines à partir d'hydrocarbures saturés inférieurs en $C_5$, seuls ou en mélange, notamment l'éthane et le propane. La nitration peut être conduite en présence d'oxygène sous forme d'air, d'air suroxygéné ou d'oxygène pur. Pour orienter la fabrication vers un spectre donné de nitroparaffines on a souvent avantage à conduire la nitration en présence d'un agent actif porteur d'un groupement NO ou $NO_2$ facilement transférable, tels le 2-nitropropane et le nitroéthane seuls ou en mélange, qui peuvent être des produits de recyclage de la réaction; on peut agir également en présence d'adjuvants.

Les variations du rapport molaireagent actif/agent nitrant donnent une grande souplesse d'adaptation du procédé à la demande du marché. Et ce rapport est ajusté en fonction du spectre attendu des nitroparaffines.

L'agent nitrant est choisi parmi le peroxyde d'azote, l'acide nitrique, seuls ou en mélange, ou tout agent porteur d'un groupe NO et $NO_2$ facilement transférable.

D'autre part, la conduite de la nitration en présence d'un ou plusieurs gaz inertes, vis à vis de la réaction et des produits réactés, peut faciliter la récupération de l'hydrocarbure résiduel en sortie de la nitration. Ce gaz inerte peut être choisi parmi l'azote, l'oxyde de carbone, le dioxyde de carbone, l'hydrogène, le méthane, l'argon ou un mélange de ces gaz.

De plus, la température de réaction dans les limites indiqués précédemment, assurant pour une pression donnée la nitration en phase vapeur homogène, est choisie en fonction de l'hydrocarbure ou du mélange d'hydrocarbures traité, des adjuvants recyclés, des rapports des constituants du mélange des réactants à l'entrée de la zone réactionnelle, pour obtenir un mélange de nitroparaffines d'un spectre déterminé.

Les gaz sortant de la zone réactionnelle de nitration sont refroidis aussi rapidement que possible et à température aussi basse que possible mais compatible avec la pression en évitant toute condensation de l'hydrocarbure n'ayant pas réagi. Après cette trempe on obtient deux phases: la phase liquide et la phase gazeuse. La phase liquide qui est extraite de la boucle contient notamment les nitroparaffines auxquelles on fait subir un ensemble de traitements de purification avant distillation; tandis que la phase gazeuse est soumise à un premier traitement de lavage à l'eau, qui assure l'élimination des produits oxydés: aldéhydes et cétones et de divers produits nitrilés. Elle est ensuite soumise à un lavage par une solution de sulfate métallique choisi parmi les sulfates des métaux suivants fer Fe++, cobalt Co++, cuivre Cu++, en vue de l'absorption du monoxyde d'azote. La majeure partie de cette phase gazeuse épurée est recyclée dans la boucle de nitration après réchauffage et recompression à la pression de nitration.

Selon le procédé on extrait de la boucle de nitration les produits fabriqués utilisables sous forme liquide et les produits gazeux qui ne peuvent pas être recyclés sous peine de voir s'enrichir la boucle en gaz indésirables. Une purge continue de déconcentration permet l'élimination du monoxyde de carbone, du dioxyde de carbone et de l'azote introduits ou formés dans la réaction. Afin de réduire au minimum les pertes de l'unité de fabrication de nitroparaffines, cette purge représente 10 à 20% de la quantité du gaz de la boucle de nitration. Cette partie de la phase gazeuse constituant la purge continue de déconcentration est soumise sous pression à une épuration secondaire comprenant un traitement de récupération du ou des hydrocarbures dans un solvant aliphatique de masse moléculaire comprise entre 100 et 200, tel un hydrocarbure saturé supérieur à $C_6$ puis à un lavage par une solution contenant des sulfates des métaux précédemment cités en vue de procéder à la récupération du monoxyde d'azote. Les solutions ayant servi à ces lavages absorbants sont régénérées et les hydrocarbures d'une part, et le monoxyde d'azote d'autre part après traitement d'oxydation en peroxyde d'azote sont recyclés.

Au titre d'agent oxydant, en présence duquel on met en œuvre la nitration, l'air ou l'oxygène peuvent être employés. Toutefois l'oxygène conduit à la solution la plus élégante au point de vue technique. En effet la présence de quantités importantes d'azote ou bien conduit à des pertes appréciables d'hydrocarbures entrainés avec la purge gazeuse ou nécessite un système de récupération des hydrocarbures qui peut être complexe.

La disposition d'oxygène permet aisément de produire le peroxyde d'azote consommé dans la réaction de nitration et en outre de réoxyder le monoxyde d'azote récupéré et régénéré après la nitration.

Le monoxyde d'azote correspondant au peroxyde d'azote consommé dans la réaction de nitration est fabriqué par oxydation d'ammoniac par l'oxygène en présence de vapeur d'eau; après condensation et lavage, le monoxyde d'azote formé est additionné au monoxyde d'azote extrait de la boucle principale de nitration par le lavage approprié décrit de la phase gazeuse par des sulfates métalliques puis régénérés. La totalité du monoxyde d'azote est alors oxydée sous la pression de la boucle de nitration avec de l'oxygène en phase concentrée dans un réacteur spécialement adapté pour assurer la maîtrise et le contrôle de la réaction en évitant tout emballement de celle-ci, et la récupération des calories de cette réaction très exothermique.

Il est avantageux de conduire l'oxydation de l'ammoniac par l'oxygène en présence de vapeur d'eau catalysée sur toiles de platine à une température comprise entre 780 et 900°C, préférentiellement entre 820 et 850°C. Ce type d'oxydation produit du monoxyde d'azote avec un rendement moyen compté en azote de 80 à 90%. Après condensation de l'eau, le monoxyde d'azote est comprimé à la pression de la boucle et est oxydé en présence d'oxygène en excès pour fournir à la boucle le peroxyde d'appoint en même temps que l'oxygène. Cette oxydation du monoxyde d'azote en peroxyde d'azote se fait à température comprise entre 750°C et la température ambiante et préférentiellement entre 550 et 100°C dans un réacteur spécialement adapté en vue de l'élimination rapide de la chaleur de réaction. Ce réacteur peut-être une chaudière à effet rapide possédant des introductions multiples d'oxygène dans le monoxyde d'azote.

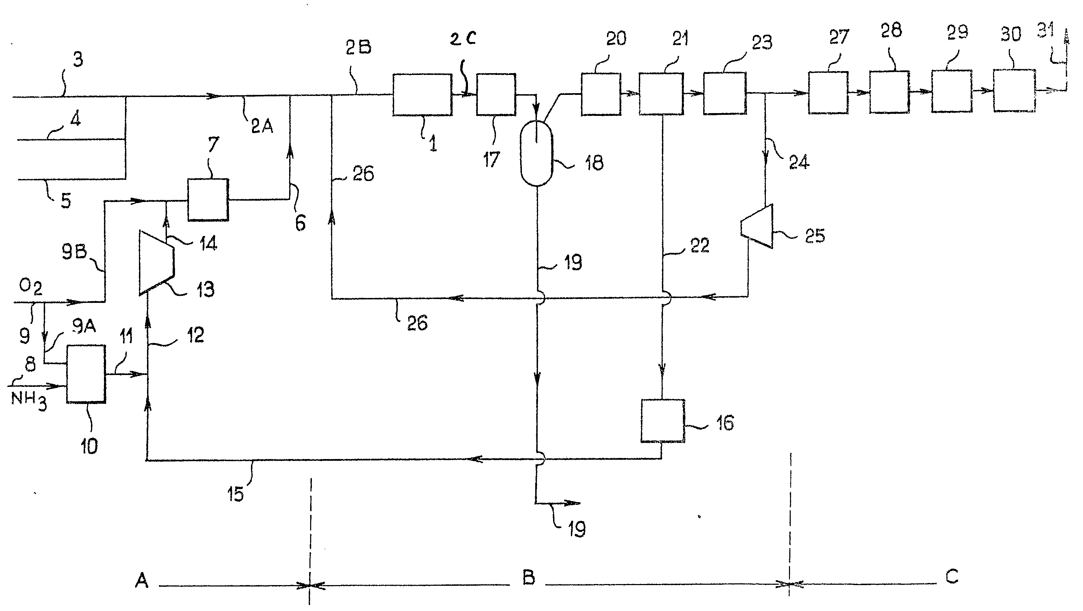

Une installation de fabrication de nitroparaffines adaptée à la mise en œuvre du procédé de l'invention est représentée sur la figure du dessin annexé. Elle comprend une boucle principale de nitration (B) constituée par un réacteur de nitration (1), un récupération thermique de la chaleur réactionnelle (17), un séparateur d'extraction (18) des produits condensés après nitration retirés par la canalisation (19), et des effluents gazeux, intercalé sur la ligne de nitration entre le récupérateur thermique et les dispositifs d'épuration des gaz effluents (20 et 23) et de récupération du monoxyde d'azote (21), un circuit d'extraction (24) de la majeure partie de la phase gazeuse effluente épurée et débarrassée du monoxyde d'azote, sur lequel est intercalé un surpresseur (25) qui comprime les gaz n'ayant pas réagi sous la pression de la boucle et les renvoie par la canalisation (26) puis (2B) au réacteur (1).

L'installation comprend une ligne de purge continue (C) sur laquelle sont intercalés les dispositifs 27, 28, 29 et 30 respectivement dispositif de lavage du gaz de purge par un solvant aliphatique (27), dispositif de récupération de l'hydrocarbure n'ayant pas réagi (28) qui sera recyclé et réintroduit dans l'installation par la canalisation (3), dispositif de lavage des gaz par une solution de sulfate métallique (29) et dispositif de récupération du monoxyde d'azote (30) et après décompression les gaz sortant par le circuit (31) peuvent être brûlés dans une torche.

L'installation comprend un système d'alimentation (A) des divers constituants du mélange réactionnel; l'hydrocarbure est introduit par la canalisation (3), les adjuvants par (4) et les produits recyclés par (5), ces constituants sont réunis dans la canalisation (2A) et par (2B) entrent dans le

3

réacteur (1). L'installation comprend en outre le circuit de récupération du monoxyde d'azote récuéré à partir de la boucle principale à partir du dispositif (21), ce monoxyde récupéré est véhiculé à travers la canalisation (22), jusqu'au dispositif de régénération (16) par les canalisation (15) et (12) jusqu'au surpresseur (13) par (14) le monoxyde comprimé sous la pression de la boucle principale est oxydé dans le réacteur d'oxydation (7) et le peroxyde d'azote formé est réintroduit dans la boucle principale de nitration par la canalisation (6) puis par (2B) jusqu'au réacteur de nitration.

Optionnellement, l'installation peut comprendre un réacteur d'oxydation d'ammoniac (10), dans lequel l'ammoniac et l'oxygène sont respectivement introduits par les canalisations (8) et (9A), le monoxyde d'azote formé extrait par la canalisation (11) est additionné au monoxyde d'azote régénéré dans la canalisation (12). L'oxygène présent dans le réacteur de nitration (1) est véhiculé par la canalisation (9B) jusqu'au réacteur d'oxydation du monoxyde d'azote (7), d'où par la canalisation (6) en même temps que le peroxyde d'azote fabriqué et régénéré, il est introduit dans le circuit de nitration.

Il est donné ci-après un exemple qui illustre le procédé de l'invention mis en œuvre dans l'installation telle que décrite.

### Exemple

On réalise la nitration d'un mélange éthane-propane, par le peroxyde d'azote, en présence d'oxygène, avec recyclage de 2-nitropropane, également en présence de gaz inerte composé de monoxyde et de dioxyde de carbone, et d'adjuvant. La pression dans la boucle principale de nitration est de 10 bars et la température de nitration de 332°C, temps de contact 5,5 secondes.

La nitration est conduite avec recyclage des gaz n'ayant pas réagi, récupération du monoxyde d'azote, transformation en peroxyde d'azote et appoint de peroxyde d'azote provenant d'une unité d'oxydation d'ammoniac par de l'oxygène, l'appoint d'oxygène est constitué par l'oxygène introduit en excès dans l'oxydation du monoxyde d'azote en peroxyde d'azote.

Dans cette marche de l'installation de l'unité de nitration on procède à une purge continue de 20% de la quantité du gaz de la boucle de nitration. On procède également à un appoint continu en propane-éthane, 2-nitropropane, adjuvant, peroxyde d'azote et oxygène.

Les compositions pondérales des différents constituants, réactants et effluents en divers points de l'installation sont consignées dans le tableau ci-après.

Les composants sont désignés par les abréviations suivantes:

$C_2H_6$ éthane; $C_3H_8$ propane; $O_2$ oxygène; $NO_2$ peroxyde d'azote; $NO$ monoxyde d'azote; $NH_3$ ammoniac; $CO$ monoxyde de carbone; $CO_2$ dioxyde de carbone; $2 NC_3$ 2-nitropropane; $NC_1$ nitrométhane; $NC_2$ nitroéthane; $1 NC_3$ 1-nitropropane.

La marche de l'installation a été suivie et les compositions indiquées pour les canalisations 2B, 2C, 19, 24, 31, 8, 9 et 2A de la figure du dessin annexé et les quantités sont données en kilogrammes

4

Tableau

| Composants | 2 B | 2 C | 19 | 24 | 31 | 8 | 9 | 2 A |
|---|---|---|---|---|---|---|---|---|
| $C_2H_6$ | 1070 | 1000 | | 804 | 2,2 | | | 105 |
| $C_3H_8$ | 1181 | 917 | | 726 | 0,6 | | | 212 |
| $O_2$ | 92 | 0 | | | | | 535 | |
| $NO_2$ | 448 | 0 | | | | | | |
| NO | 0 | 158 | | 37 | 0,4 | | | |
| $NH_3$ | 0 | | | | | 107 | | |
| CO | 213 | 262 | | 213 | 44,8 | | | |
| $CO_2$ | 429 | 584 | | 429 | 5,5 | | | |
| 2 $NC_3$ | 133 | 171 | 165 | | | | | 133 |
| $NC_1$ | | 61 | 56 | | | | | |
| $NC_2$ | | 37 | 36 | | | | | |
| 1 $NC_3$ | | 22 | 21 | | | | | |
| adjuvant (Ethanal) | 7 | | | | | | | 7 |

## Revendications

1 — Procédé de fabrication de nitroparaffines par nitration en phase gazeuse homogène d'un hydrocarbure saturé inférieur à $C_5$, tel l'éthane et le propane seuls ou en mélange, par un agent nitrant tel le peroxyde d'azote, l'acide nitrique seuls ou en mélange, selon lequel la nitration est conduite en présence d'un agent oxydant tel l'oxygène et éventuellement de produits de recyclage de la nitration tels le 2-nitropropane et le nitroéthane seuls ou en mélange, éventuellement en présence de gaz inerte vis à vis du mélange réactionnel, et sous une pression de 1 à 30 bars, préférentiellement de 8 à 20 bars, caractérisé en ce que les diverses opérations constituant l'ensemble du procédé en ce que les diverses opérations constituant l'ensemble du procédé sont conduites sous une pression unique, la réaction de nitration étant mise en œuvre en boucle fermée sous cette dite pression avec séparation des effluents réactionnnels en phase gazeuse et en phase liquide contenant notamment les nitroparaffines produites, la phase gazeuse étant soumise à un traitement d'épuration sous la dite pression, puis la majeure partie de cette phase gazeuse épurée et réchauffée étant recyclée dans la boucle de nitration sous la pression de celle-ci, il est procédé à une purge continue de déconcentration de l'autre partie de la phase gazeuse soumise à des opérations d'épuration et de récupération de l'hydrocarbure et du monoxyde d'azote, et avec un appoint continu en hydrocarbure d'une part, et agent nitrant d'autre part dans la boucle de nitration sous la pression de celle-ci.

2 — Procédé de fabrication de nitroparaffines selon la revendication 1, caractérisé en ce que le monoxyde d'azote extrait de la boucle de nitration par lavage de la phase gazeuse par une solution de sulfate métallique est régénérée, est comprimé sous la pression de la boucle de nitration, puis sous cette pression oxydé en peroxyde d'azote et réintroduit dans la boucle de nitration.

3 — Procédé de fabrication de nitroparaffines selon la revendication 1, caractérisé en ce que l'épuration primaire de la phase gazeuse comprend au moins un traitement à l'eau et un lavage par une solution de sulfate métallique choisi parmi les sulfates de fer, de cobalt ou de cuivre.

4 — Procédé de fabrication de nitroparaffines selon la revendication 1, caractérisé en ce que la partie de la phase gazeuse constituant la purge continue de déconcentration est soumise sous pression à une épuration secondaire comprenant un traitement par un solvant aliphatique de masse moléculaire comprise entre 100 et 200 puis un lavage par une solution de sulfate métallique choise parmi le sulfate de fer, de cobalt, ou de cuivre.

5 — Procédé de fabrication de nitroparaffines selon la revendication 1, caractérisé en ce que la nitration est conduite en régime thermique progressif et régulier avec une température de sortie des effluents en fin de nitration inférieure à 400°C.

6 — Procédé de fabrication de nitroparaffines selon la revendication 1, quand l'agent nitrant est le peroxyde d'azote, caractérisé en ce que l'appoint en peroxyde d'azote provient d'une oxydation d'ammoniac par l'oxygène; le monoxyde d'azote ainsi produit et le monoxyde d'azote régénéré sont oxydés sous la pression de la boucle de nitration en présence d'oxygène en excès, et le peroxyde d'azote formé introduit dans la boucle de nitration avec l'oxygène en excès.

7 — Installation de fabrication de nitroparaffines destinée à mettre en œuvre le procédé de nitration selon la revendication 1, caractérisé en ce qu'elle comprend une boucle principale (B) constituée par un réacteur de nitration (1), un récupérateur thermique de la chaleur réactionnelle (17), des dispositifs d'épuration des gaz effluents (20 et 23), et de récupération du monoxyde d'azote (21), un surpresseur de réintroduction (25) à l'entrée du réacteur des gaz n'ayant pas réagi, un séparateur d'extraction (18) des produits condensés après nitration, intercalé sur la ligne de nitration entre le récupérateur thermique (17) et les dispositifs d'épuration (20 et 23); elle comprend une purge continue de déconcentration (C) après les dispositifs d'épuration, comprenant elle-même des dispositifs de traitement d'épuration gaz de purge (27) et de soutirage des produits récupérés (28); elle comprend en outre, le circuit d'alimentation (A) des divers constituants du mélange réactionnel et la boucle de l'agent nitrant, constituée par un circuit de récupération du monoxyde d'azote à partir de la phase gazeuse épurée (22), un dispositif de régénération de ce monoxyde d'azote (16), un surpresseur du monoxyde d'azote (13), un réacteur d'oxydation (7) du monoxyde d'azote en peroxyde d'azote et un circuit d'introduction du peroxyde d'azote dans la boucle principale (6 et 2B).

8 — Installation de fabrication de nitroparaffines selon la revendication 7, caractérisée en ce qu'un réacteur d'oxydation d'ammoniac (10) en monoxyde d'azote et un circuit d'alimentation en oxygène (9B) sont associés à la boucle de l'agent nitrant.

## Patentansprüche

1. Verfahren zur Herstellung von Nitroparaffinen durch Nitrierung eines gesättigten Kohlenwasserstoffes mit weniger als 5 Kohlenstoffatomen, wie Äthan und/oder Propan, in homogener Gasphase mit einem Nitriermittel, wie Stickstoffperoxid und/oder Salpetersäure, wobei die Nitrierung in Gegenwart eines Oxidationsmittels, wie Sauerstoff, und gegebenenfalls von Rezyklierungsprodukten der Nitrierung, wie 2-Nitropropan und/oder Nitroäthan, gegebenenfalls in Gegenwart von gegenüber dem Reaktionsgemisch inertem Gas und unter einem Druck von 1 bis 30 bar, vorzugsweise 8 bis 20 bar, durchgeführt wird, dadurch gekennzeichnet, daß die verschiedenen, die Gesamtheit des Verfahrens bildenden Operationen unter einem einzigen Druck durchgeführt werden, wobei die Nitrierungreaktion in einem geschlossenen Kreislauf unter diesem besagten Druck unter Trennung der Reaktionsausläufe in eine Gasphase und eine besonders die erzeugten Nitroparaffinen enthaltende flüssige Phase durchgeführt wird, die Gasphase einer Reinigungsbehandlung unter diesem Druck unterzogen wird und dann der größere Teil dieser gereinigten und erwärmten Gasphase in den Nitrierkreislauf unter dem Druck desselben zurückgeführt wird, und daß zur Konzentrationsminderung eine fortgesetzte Abgabe des anderen Teils der Gasphase, die den Operationen einer Reinigung und Wiedergewinnung von Kohlenwasserstoff und Stickstoffmonoxid unterzogen wurde, vorgenommen wird, wobei fortgesetzt einerseits Kohlenwasserstoff und andererseits Nitriermittel dem Nitrierkreislauf bei dessen Druck zugesetzt werden.

2. Verfahren zur Herstellung von Nitroparaffinen nach Anspruch 1, dadurch gekennzeichnet, daß das aus dem Nitrierkreislauf durch Waschen der Gasphase mit einer Metallsulfatlösung extrahierte Stickstoffmonoxid regeneriert, auf den Druck des Nitrierkreislaufes komprimiert, sodann bei diesem Druck zu Stickstoffperoxid oxidiert und in den Nitrierkreislauf wieder eingeführt wird.

3. Verfahren zur Herstellung von Nitroparaffinen nach Anspruch 1, dadurch gekennzeichnet, daß die primäre Reinigung der Gasphase wenigstens eine Behandlung mit Wasser und eine Waschung mit einer Lösung von Metallsulfat aus der Gruppe der Sulfate von Eisen, Kobalt oder Kupfer umfaßt.

4. Verfahren zur Herstellung von Nitroparaffinen nach Anspruch 1, dadurch gekennzeichnet, daß der Teil der Gasphase, der zur Konzentrationverminderung fortgesetzt abgegeben wird, unter Druck einer sekundären Reinigung unterzogen wird, die eine Behandlung mit einem aliphatischen Lösungsmittel vom Molekulargewicht zwischen 100 und 200 und sodann eine Waschung mit einer Lösung von Metallsulfat aus der Gruppe der Sulfate von Eisen, Kobalt oder Kupfer umfaßt.

5. Verfahren zur Herstellung von Nitroparaffinen nach Anspruch 1, dadurch gekennzeichnet, daß die Nitrierung in einem voranschreitenden und gleichmäßigen Wärmebereich mit einer Temperatur des Ausgangs der Ausläufe zur Nitrierung unterhalb 400°C durchgeführt wird.

6. Verfahren von Nitroparaffinen nach Anspruch 1, wenn das Nitriermittel Stickstoffperoxid ist, dadurch gekennzeichnet, daß Stickstoffperoxid zugegeben wird, das aus einer Oxidation von Ammoniak mit Sauerstoff stammt, daß das auf diese Weise erzeugte Stickstoffmonoxid und das regenerierte Stickstoffmonoxid unter dem Druck des Nitrierkreislaufs in Gegenwart von überschüssigem Sauerstoff oxidiert werden und daß das gebildete Stickstoffperoxid mit überschüssigem Sauerstoff in den Nitrierkreislauf eingeführt wird.

7. Vorrichtung zur Herstellung von Nitroparaffinen unter Anwendung des Nitrierverfahrens nach Anspruch 1, gekennzeichnet durch einen Hauptkreislauf (B), der aus einem Nitrierungsreaktor (1), einer Wärmewiedergewinnungseinrichtung für Reaktionswärme (17), Einrichtungen zur Reinigung von Gasausflüssen (20 und 23) und zur Wiedergewinnung von Stickstoffmonoxid (21), einem Überdruckgebläse (25) für die Wiedereinführung von unumgesetzten Gasen am Eintritt des Reaktors und einer Trenneinrichtung (18) für die Gewinnung von nach der Nitrierung kondensierten Produkten, die in die Nitrierungslinie zwischen der Wärmegewinnungseinrichtung (17) und den Reinigungseinrichtungen (20 und 23) eingeschaltet ist, besteht, durch eine Einrichtung für kontinuierlichen Gasabzug (C) hinter den Reinigungseinrichtungen, die selbst Einrichtungen zur Reinigungsbehandlung des abgezogenen Gases (27) und zur Entfernung wiedergewonnener Produkte (28) einschließt, und durch einen Beschickungskreislauf (A) für die verschiedenen Bestandteile des Reaktionsgemisches und einen Kreislauf des Nitriermittels, der aus einem Kreislauf für die Wiedergewinnung von Stickstoffmonoxid aus der gereinigten Gasphase (22), einer Einrichtung zur Regenerierung dieses Stickstoffmonoxids (16), einem Überdruckgebläse für Stickstoffmonoxid (13), einem Reaktor (7) zur Oxidation von Stickstoffmonoxid zu Stickstoffperoxid und einem Kreislauf zur Einführung von Stickstoffperoxid in den Hauptkreislauf (6 und 2B) besteht. .

8. Vorrichtung zur Herstellung von Nitroparaffinen nach Anspruch 7, dadurch gekennzeichnet, daß ein Reaktor (10) für die Oxidation von Ammoniak zu Stickstoffmonoxid und eine Beschickungsleitung (9B) für Sauerstoff mit dem Kreislauf des Nitriermittels verbunden sind.

## Claims

1. A process for the production of nitroparaffins by nitration in the homogeneous gaseous phase of a staurated lower hydrocarbon below $C_5$, such as ethane and propane alone or in admixture, using a nitrating agent such as nitrogen peroxide or nitric acid alone or in admixture according to which process the nitration is carried out in the presence of an oxidising agent such as oxygen and possibly of recycled nitration products, such as 2-nitropropane or nitroethane alone or in admixture, possibly in the pressence of a gas inert with respect to the reaction mixture, and under a pressure of 1 to 30 bars, preferably 8 to 20 bars, characterised in that the several steps of the process are carried out at the same pressure, the nitrating reaction being carried out in a closed loop under the said pressure with separation of the reaction effluents into a gaseous phase and a liquid phase containing the nitroparaffins produced, the gaseous phase being subjected to a purification treatment under the said pressure, whereupon the major proportion of this purified and reheated gaseous phase is recycled to the nitration loop under the pressure of the latter, that a continuous purification is undertaken for dilution of the other part of the gaseous phase submitted to purification and for the recovery of the hydrocarbon and of nitrogen monoxide, and with continuous addition of hydrocarbon on the one hand, and of the nitrating agent on the other hand, to the nitration loop under the pressure of the latter.

2. A process for the production of nitroparaffins according to Claim 1, characterised in that the nitrogen monoxide extracted from the nitration loop by scrubbing of the gaseous phase with a solution of metal sulphate is regenerated, compressed to the pressure of the nitration loop and then oxidised to nitrogen peroxide under this pressure and returned to the nitration loop.

3. A process for the production of nitroparaffins according to Claim 1, characterised in that the initial purification of the gaseous phase comprises at least one treatment with water and washing with a solution of a metal sulphate selected from the sulphates of iron, cobalt and copper.

4. A process for the production of nitroparaffines according to Claim 1, characterised in that the part of the gaseous phase forming the continuous purification is exposed under pressure to a secondary purification comprising treatment with an aliphatic solvent having a molecular weight between 100 and 200 followed by washing with a metal sulphate selected from the sulphates of iron, cobalt and copper.

5. A process for the production of nitroparaffins according to Claim 1, characterised in that the nitration is carried out in an operation having a progressive and uniform thermal balance, with the effluents at the conclusion of the nitration having an emergent temperature below 400° C.

6. A process for the production of nitroparaffins according to Claim 1, in which the nitrating agent is nitrogen peroxide, characterised in that the addition of nitrogen peroxide is derived from the oxidation of ammonia with oxygen; the nitrogen monoxide thus produced, and the regenerated nitrogen monoxide are oxidised at the pressure of the nitration loop in the presence of an excess of oxygen, and the nitrogen peroxide formed is introduced into the nitration loop together with the excess of oxygen.

7. Apparatus for the production of nitroparaffins according to the nitration process of Claim 1, characterised in that it comprises a principal loop (B) formed by a nitration reactor (1), means for recovering reaction heat (17), devices (20 and 23) for the purification of effluent gases and for the recovery of nitrogen monoxide (21), a blower (25) for reintroduction at the inlet of the reactor of the unreacted gases, a separator for extraction (18) of the condensed products after nitration disposed in the nitration line between the heat recovery means (17) and the purifying devices (20 and 23); which comprises a continuous dilution purifier (C) following the purifying devices, and also comprises devices for the purification of puring gas (27) and for the withdrawal of the recovered products (28);

and additionally comprises the circuit (A) for supplying the several components of the reaction mixture and the loop of the nitrating agent, formed by a circuit for the recovery of the nitrogen monoxide from the purified gaseous phase (22), a device for regeneration of the nitrogen monoxide (16), a blower (13) for the nitrogen monoxide, a reactor (7) for oxidation of the nitrogen monoxide to nitrogen peroxide, and a circuit for introducing nitrogen peroxide into the principal loop (6 and 2B).

8. Apparatus for the production of nitroparaffins according to Claim 7, characterised in that a reactor for oxidation of ammonia (10) to nitrogen monoxide, and an oxygen supply circuit (9B) are associated with the loop of the nitrating agent.

8

0 017 560